(19) 

Europäisches
Patentamt

European
Patent Office

Office européen
des brevets

(11)  **EP 4 721 583 A1**

(12)  **EUROPEAN PATENT APPLICATION**
published in accordance with Art. 153(4) EPC

(43) Date of publication:
**08.04.2026  Bulletin 2026/15**

(21) Application number: **24815351.2**

(22) Date of filing: **23.05.2024**

(51) International Patent Classification (IPC):
*A23L 33/105* (2016.01)    *A23F 3/30* (2006.01)
*A23L 2/52* (2006.01)    *A61K 35/744* (2015.01)
*A61K 36/82* (2006.01)    *A61P 43/00* (2006.01)
*C12N 1/20* (2026.01)

(52) Cooperative Patent Classification (CPC):
A23F 3/30; A23L 2/52; A23L 33/105; A61K 35/744;
A61K 36/82; A61P 43/00; C12N 1/20

(86) International application number:
**PCT/JP2024/018958**

(87) International publication number:
**WO 2024/247867 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.05.2023  JP 2023088180**

(71) Applicant: **AutoPhagyGO Inc.
Suita-shi, Osaka 565-0871 (JP)**

(72) Inventors:
• **TATEBE, Hisashi
Suita-shi, Osaka 565-0871 (JP)**

• **FUKUDA, Kanako
Suita-shi, Osaka 565-0871 (JP)**
• **SHOJI, Shisako
Suita-shi, Osaka 565-0871 (JP)**
• **SHOSHIHARA, Masako
Suita-shi, Osaka 565-0871 (JP)**
• **ISHIDO, Miwako
Suita-shi, Osaka 565-0871 (JP)**
• **IKAWA, Keitaro
Anan-shi, Tokushima 771-5173 (JP)**

(74) Representative: **WSL Patentanwälte
Partnerschaft mbB
Kaiser-Friedrich-Ring 98
65185 Wiesbaden (DE)**

(54)  **AUTOPHAGY ACTIVATOR, HYALURONIC ACID PRODUCTION PROMOTER, AND
ANTIOXIDANT**

(57)    The present disclosure provides a new autophagy activator.
    Provided are an autophagy activator, a hyaluronic acid production promoter, and an antioxidant that contain a lactic acid fermentation product obtained by fermenting a tea plant-derived raw material with lactic acid bacteria.

[FIG.1]

AWA BANCHA (BEADS): EXTRACT FROM CRUSHED TEA LEAVES OF AWA BANCHA

EGFP AMOUNT /mRFP AMOUNT RELATIVE TO NEGATIVE CONTROL — CONCENTRATION IN MEDIUM (%)

EP 4 721 583 A1

## Description

Technical Field

The present disclosure relates to an autophagy activator, a hyaluronic acid production promoter, and an antioxidant.

Background Art

Autophagy is one of intracellular protein degradation systems possessed by eukaryotes. Specifically, first, an isolation membrane that has appeared in a cell surrounds a part of the cytoplasm or an intracellular organelle to form an autophagosome. Subsequently, a lysosome fuses with the autophagosome to form an autolysosome. In the autolysosome, encapsulated proteins and the like are decomposed by hydrolyzing enzymes in the lysosome. From such a mechanism, for example, an effect of removing aggregated proteins in neurodegenerative diseases such as Parkinson's disease is expected (Patent Literature 1). As described above, the usefulness of physiological functions by autophagy is being found, and development of substances having autophagy activity is desired.

Citation List

Patent Literature

Patent Literature 1: JP 2019-94303 A

Summary of Invention

Technical Problem

An object of the present disclosure is to provide a new autophagy activator.

Solution to Problem

The present inventors have conducted various studies and found that a specific material has excellent autophagy activity. The present inventors have also found that this specific material has a hyaluronic acid production promoting activity and an antioxidant activity, and have completed the present disclosure.

The present disclosure (1) is an autophagy activator comprising a lactic acid fermentation product (i.e., lactic acid bacteria fermentation product) obtained by fermenting a tea plant-derived raw material with lactic acid bacteria.

The present disclosure (2) is the autophagy activator according to the disclosure (1), wherein the lactic acid fermentation product is obtained by submerging the tea plant-derived raw material after boiling and/or steaming with the lactic acid bacteria to perform lactic acid fermentation.

The present disclosure (3) is the autophagy activator according to the disclosure (1) or (2), wherein the tea plant-derived raw material is a tea leaf.

The present disclosure (4) is the autophagy activator according to any one of the disclosures (1) to (3), wherein the lactic acid bacteria include at least one selected from Lactobacillus pentosus and Lactobacillus plantarum.

The present disclosure (5) is the autophagy activator according to any one of the disclosures (1) to (4), wherein the lactic acid fermentation product is Awa Bancha.

The present disclosure (6) is a food, beverage, supplement, cosmetic, pharmaceutical or quasi-pharmaceutical product comprising the autophagy activator according to any one of the disclosures (1) to (5).

The present disclosure (7) is a cosmetic for skin elasticity improvement, moisture-retention improvement, wrinkle prevention, anti-aging, or age-defying, the cosmetic comprising the autophagy activator according to any one of the disclosures (1) to (5).

The present disclosure (8) is a supplement for anti-aging, age-defying or lifestyle-disease prevention, the supplement comprising the autophagy activator according to any one of the disclosures (1) to (5).

The present disclosure (9) is a hyaluronic acid production promoter comprising a lactic acid fermentation product (i.e., lactic acid bacteria fermentation product) obtained by fermenting a tea plant-derived raw material with lactic acid bacteria.

The present disclosure (10) is the hyaluronic acid production promoter according to the disclosure (9), wherein the lactic acid fermentation product is obtained by submerging the tea plant-derived raw material after boiling and/or steaming with the lactic acid bacteria to perform lactic acid fermentation.

The present disclosure (11) is the hyaluronic acid production promoter according to the disclosure (9) or (10), wherein the tea plant-derived raw material is a tea leaf.

The present disclosure (12) is the hyaluronic acid production promoter according to any one of the disclosures (9) to (11), wherein the lactic acid bacteria include at least one selected from Lactobacillus pentosus and Lactobacillus plantarum.

The present disclosure (13) is the hyaluronic acid production promoter according to any one of the disclosures (9) to (12), wherein the lactic acid fermentation product is Awa Bancha.

The present disclosure (14) is a food, beverage, supplement, cosmetic, pharmaceutical or quasi-pharmaceutical product comprising the hyaluronic acid production promoter according to any one of the disclosures (9) to (13).

The present disclosure (15) is a cosmetic for skin elasticity improvement, moisture-retention improvement, wrinkle prevention, anti-aging, or age-defying, the cosmetic comprising the hyaluronic acid production promoter according to any one of the disclosures (9) to (13).

The present disclosure (16) is a supplement for anti-aging, age-defying or lifestyle-disease prevention, the supplement comprising the hyaluronic acid production promoter according to any one of the disclosures (9) to (13).

The present disclosure (17) is an antioxidant comprising a lactic acid fermentation product (i.e., lactic acid bacteria fermentation product) obtained by fermenting a tea plant-derived raw material with lactic acid bacteria.

The present disclosure (18) is the antioxidant according to the disclosure (17), wherein the lactic acid fermentation product is obtained by submerging the tea plant-derived raw material after boiling and/or steaming with the lactic acid bacteria to perform lactic acid fermentation.

The present disclosure (19) is the antioxidant according to the disclosure (17) or (18), wherein the tea plant-derived raw material is a tea leaf.

The present disclosure (20) is the antioxidant according to any one of the disclosures (17) to (19), wherein the lactic acid bacteria include at least one selected from Lactobacillus pentosus and Lactobacillus plantarum.

The present disclosure (21) is the antioxidant according to any one of the disclosures (17) to (20), wherein the lactic acid fermentation product is Awa Bancha.

The present disclosure (22) is a food, beverage, supplement, cosmetic, pharmaceutical or quasi-pharmaceutical product comprising the antioxidant according to any one of the disclosures (17) to (21).

The present disclosure (23) is a cosmetic for skin elasticity improvement, moisture-retention improvement, wrinkle prevention, anti-aging, or age-defying, the cosmetic comprising the antioxidant according to any one of the disclosures (17) to (21).

The present disclosure (24) is a supplement for anti-aging, age-defying or lifestyle-disease prevention, the supplement comprising the antioxidant according to any one of the disclosures (17) to (21).

Advantageous Effects of Invention

According to the present disclosure, a new autophagy activator can be provided.

Brief Description of Drawings

[Fig. 1] Fig. 1 is a graph showing a fluorescence amount ratio, expressed as a relative value when EGFP amount/mRFP amount of negative control is set to 1, in HeLa cells upon addition of the lactic acid fermentation product according to Example 1 at various concentrations.

[Fig. 2] Fig. 2 is a graph showing a fluorescence amount ratio, expressed as a relative value when EGFP amount/mRFP amount of negative control is set to 1, in HeLa cells upon addition of the lactic acid fermentation product according to Example 2 at various concentrations.

[Fig. 3] Fig. 3 is a graph showing the amount (unit: $\mu$g/mL) of hyaluronic acid in a culture supernatant of normal human adult skin fibroblasts upon addition of the lactic acid fermentation product according to Example 3 at various concentrations.

[Fig. 4] Fig. 4 is a graph showing the amount (unit: $\mu$g/mL) of hyaluronic acid in a culture supernatant of normal human skin fibroblast cells upon addition of the lactic acid fermentation product according to Example 3 at various concentrations.

Description of Embodiments

Hereinafter, one embodiment of the present disclosure will be described in detail. Note that the present disclosure is not limited to the following embodiment.

In the present description, when a plurality of upper limit values and a plurality of lower limit values are described separately, all numerical ranges that can be set by freely combining these upper limit values and lower limit values are described.

(Autophagy Activator)

The term "autophagy" as used herein refers to a system in which intracellular organelles and proteins are degraded in cells via autophagosome formation. In addition, "autophagy activation" or "autophagy is activated" refers to a state in which a recycling mechanism of intracellular proteins is activated by induction or promotion of autophagy in cells. Specifically, "autophagy activation" or "autophagy is activated" refers to a state in which at least one of the following processes is induced or promoted: formation of an isolation membrane, elongation of an isolation membrane, formation of an autophagosome, and fusion of an autophagosome and a lysosome.

The activity of autophagy can be confirmed by a conventionally known autophagy activity measuring method. Examples of such an autophagy activity measuring method include a method for detecting an autophagy marker protein. Examples of the autophagy marker protein include LC3 (e.g., rat microtubule-associated protein 1 light chain 3). LC3 has two forms, LC3-I and LC3-II. It is known that LC3 exists as LC3-I when it exists in cytoplasm, and LC3-I is converted to lipidated LC3-II upon formation of autophagosomes and is incorporated into inner and outer membranes of autophagosomes. Thus, by detecting LC3-I and LC3-II and measuring the conversion rate to LC3-II, autophagy activity can be confirmed (see, Kabeya Y., Mizushima N., Ueno T., Yamamoto A., Kirisako T., Noda T., Kominami E., Ohsumi Y., Yoshimori T.: LC3, a mammalian homologue of yeast Apg8p, is localized in autophagosome membranes after processing. The EMBO Journal, 19:5720-5728 (2000)).

The activity of autophagy can also be confirmed by expressing tfLC3 in cells, in which two fluorescent proteins (i.e., mRFP and EGFP) are fused in tandem to LC3 (see, Kimura S., Noda T., Yoshimori T.: Dissection of the Autophagosome Maturation Process by a Novel Reporter Protein, Tandem Fluorescent-Tagged LC3. Autophagy 3:452-460

(2007)). According to this method, since EGFP disappears in lysosomal or autolysosomal environments, the lower the ratio of EGFP amount/mRFP amount in a cell, the more autophagy is activated. The amount of fluorescence in cells can be measured with, for example, a fluorescence microscope, and the amount of fluorescence can be calculated with an analysis software.

(Method for Producing Autophagy Activator)

The autophagy activator according to the present embodiment contains a lactic acid fermentation product obtained by fermenting a tea plant-derived raw material with lactic acid bacteria. Details will be described below.

First, the "tea plant-derived raw material" is not particularly limited as long as it is a part of a tea plant, and is a concept including, for example, at least one or more parts selected from a leaf, a stem, a bud, and a root of tea, or a treated product thereof. Examples of the "treatment" include processing treatments such as a kneading treatment, a drying treatment, a heating treatment (e.g., a treatment such as baking, boiling, steaming, or frying), a crushing (including pulverizing) treatment, and an extraction treatment. In these treatments, the same treatment may be repeated a plurality of times, or different treatments may be combined. Other plants (e.g., yomogi, dokudami, a vegetable, a fruit, or the like) may be used as auxiliary raw materials as long as the main raw material is a tea plant-derived raw material.

The method for producing the autophagy activator includes a step of fermenting the tea plant-derived raw material (and other auxiliary raw materials) with lactic acid bacteria. For example, "post-fermented tea" that involves microbial fermentation in the production process corresponds to the product obtained by this step, and Awa Bancha produced in Tokushima is known as a type of the post-fermented tea. According to Shota Koyama et al., "Research on microorganisms of post-fermented tea produced in Tokushima, Awa Bancha", an example of a conventional method for producing Awa Bancha is as follows. First, in early summer, all fresh leaves are stripped from tea branches using a dedicated tool, placed in a pot with about 10 times the volume of the tea leaves of water, and boiled for about several minutes to 10 minutes. Thereafter, the tea leaves are separated from the boiling liquid and kneaded with a kneading machine for producing green tea for several minutes, and the tea leaves are placed in a barrel while being pressed from above. A lid or an equivalent thereof is placed on top as an inner lid, and a stone having the same weight as the tea leaves is stacked on top of the inner lid. Furthermore, the boiling liquid of the tea leaves is poured to about 10 cm above the inner lid. At this time, an appropriate fermentation odor drifts. After fermentation, the tea leaves are loosened and thoroughly sun-dried for 1 to several days on straw mats. In addition, an example of a current method for producing Awa Bancha is a method in which tea leaves of sencha green tea plants are allowed to grow larger through and beyond early summer and then harvested, boiled in hot water, kneaded, immersed in a barrel and fermented for 2 to 3 weeks, and then dried to finalize the product. In recent years, the tea leaves are harvested with a mechanical hair clipper or the finish drying is performed with a dryer or the like. Note that the above-described examples of conventional and current production methods of Awa Bancha are merely examples, and do not limit the autophagy activator according to the present disclosure.

The lactic acid bacteria to be used for fermentation during the production are not particularly limited, but preferably include at least one selected from Lactobacillus pentosus and Lactobacillus plantarum.

(Method for Using Autophagy Activator)

The autophagy activator according to the present embodiment activates the recycling mechanism of intracellular proteins, and thus can contribute to maintenance of cell homeostasis. Thus, the autophagy activator according to the present embodiment can be used as a food, beverage, supplement, cosmetic, pharmaceutical, quasi-pharmaceutical product, or the like for humans or animals, or can be a material used by being blended into a food, beverage, supplement, cosmetic, pharmaceutical, quasi-pharmaceutical product, or the like.

When the autophagy activator according to the present embodiment is used as a supplement, pharmaceutical, or the like, or used by being blended into a supplement, pharmaceutical, or the like, the supplement or the like can be administered in any administration form. Examples of the administration form include oral administration by tablets, capsules, granules, powders, syrups, and the like, and non-oral administration by injections, suppositories, transdermal absorbents, external preparations, and the like, and oral administration is preferable.

When the autophagy activator according to the present embodiment is blended into a food, supplement, cosmetic, or the like, the blending amount of the dry extract in the preparation is 0.001% by mass or more and 5% by mass or less, preferably 0.001% by mass or more and 1% by mass or less, and more preferably 0.01% mass or more and 1% by mass or less, based on the total mass of the preparation. At this time, any pharmaceutically acceptable component can be appropriately blended as a component other than the autophagy activator according to the present embodiment. Specific examples of such a component include an excipient, a binder, an extender, a disintegrant, a surfactant, a lubricant, a dispersant, a buffer, a diluent, a preservative, a flavoring agent, and a fragrance.

(Hyaluronic Acid Production Promoter)

It has been found that the lactic acid fermentation product of the present disclosure also has an action of promoting hyaluronic acid production. Although this mechanism of action has not been clarified, it is presumed that this is a result of activation of autophagy since the lactic acid fermentation product of the present disclosure has excellent autophagy activity and it is known that the production of hyaluronic acid is promoted when autophagy is activated. As described above, the lactic acid fermentation product of the present disclosure can be used as a hyaluronic acid production promoter. However, the mechanism of action by which the lactic acid fermentation product of the present disclosure exerts the action of promoting hyaluronic acid production is not limited to that via activation of autophagy, and the hyaluronic acid production promoter of the present disclosure is not limited to one that activates autophagy. The hyaluronic acid production promoting action can be confirmed, for example, by a conventionally known hyaluronic acid measurement method as disclosed in Examples described later. The method for producing and using the hyaluronic acid production promoter is the same as the method for producing and using the autophagy activator described above.

(Antioxidant)

It has been found that the lactic acid fermentation product of the present disclosure also has an antioxidant action. Although this mechanism of action has not been clarified, it is presumed that this is a result of activation of autophagy since the lactic acid fermentation product of the present disclosure has excellent autophagy activity and it is known that the an antioxidant action is exerted when autophagy is activated. As described above, the lactic acid fermentation product of the present disclosure can also be used as an antioxidant. However, the mechanism of action by which the lactic acid fermentation product of the present disclosure exerts the antioxidant action is not limited to that via activation of autophagy, and the antioxidant of the present disclosure is not limited to one that activates autophagy. The antioxidant action can be confirmed, for example, by a conventionally known active oxygen measurement method as disclosed in Examples described later. The method for producing and using the antioxidant is the same as the method for producing and using the autophagy activator described above.

The present disclosure is not limited to the above-described embodiments, and various modifications such as design changes can be made on the basis of knowledge of those skilled in the art, and embodiments to which such modifications are made are also included in the scope of the present disclosure.

Examples

<<Preparation Examples>>

(Example 1: Awa Bancha (beads): extract from crushed tea leaves of Awa Bancha)

Dried tea leaves of Awa Bancha (trade name: Awa Bancha tea leaves/manufactured by Ikawa Fermentation Co., Ltd.) were immersed in liquid nitrogen for 15 minutes to be frozen, and then pulverized at 3000 rpm for 15 seconds using a multi beads shocker (Yasui Kikai Corporation, Multi-beads Shocker MB3000). The tea leaf powder obtained by freeze pulverization was suspended in D-PBS at 100 mg/mL, vortexed for 20 seconds, then allowed to stand on ice for 10 minutes, and then centrifuged (4°C, 400G, 5 min), and the supernatant was filtered through a filter (Merck Millipore, Mill-exSLHVR33RB $\Phi$0.45$\mu$m, PVDF, 33 mm).

(Example 2: Awa Bancha (concentrate): extract prepared by boiling Awa Bancha)

A liquid obtained by boiling 25 g of the Awa Bancha tea leaves used in Example 1 with 1 liter of hot water over low heat for 60 minutes (Ikawa Fermentation Co., Ltd.) was filtered through a filter (ADVANTEC, 13HP045AN filter unit $\Phi$0.45$\mu$m).

<<Evaluation of Autophagy Activity>>

According to the method of Bhargava et al. (Bhargava K.H. et al.: Structural basis for autophagy inhibition by the human Rubicon-Rab7 complex. Proceedings of the National Academy of Sciences of the United States of America 117:17003-17010 (2020)), a tfLC3-stably expressing cell line of cervical epithelial cancer cell HeLa cells (JCRB cell bank: JCRB9004) was obtained using retrovirus. The obtained cells were suspended in a growth medium (DMEM medium supplemented with 10% FBS and 4 mM L-glutamine (catalogue number D6546; manufactured by Sigma-Aldrich)) so as to have a cell concentration of 25000 cells/mL, and dispensed into each well of a 96-well plate (Cell Carrier 96 Ultra Black 96well, Clear Bottom, with Lid; manufactured by PerkinElmer, Inc.) in an amount of 100 $\mu$L. The 96-well plate was allowed to stand in a $CO_2$ incubator and cultured for 24 hours under the conditions of 37°C, 5%$CO_2$ + 95% air.

To each of the solutions according to Examples 1 and 2, phosphate-buffered saline (PBS) (manufactured by Nacalai Tesque, Inc.) was added as a solvent, thereby preparing a dilution series of samples. Each obtained sample solution was added to the cell culture medium of each well so as to have the final concentration shown in the horizontal axis (%) of the figure. Thereafter, the wells were allowed to stand in a $CO_2$ incubator and cultured for 24 hours under the conditions of 37°C, 5%$CO_2$ + 95% air. On the other hand, as a negative control, cells cultured in a growth medium with PBS containing no sample were cultured in another well under the same conditions as described above.

After completion of the culture, the culture solution in each well was removed, and washing was performed twice by addition and removal of phosphate buffered saline (PBS). Subsequently, PBS containing 4% paraformaldehyde (manufactured by Nacalai Tesque, Inc.) and a Hoechst dye (Hoechst33342, manufactured by DOJINDO LABORATORIES) was added to each well, and the mixture was allowed to stand for 20 minutes to fix the cells.

The supernatant in each well was removed, and the wells were washed twice with PBS. The cells were imaged using a confocal Imaging cytometer (CQ1, manufactured by Yokogawa Electric Corporation) to obtain image data including information on fluorescence intensities of EGFP and mRFP. In the fluorescence intensity measurement of EGFP, the detection bandpass filter used was a detection bandpass filter BP525/50, and the excitation wavelength was 488 nm. In the fluorescence intensity measurement of mRFP, the detection bandpass filter used was a detection bandpass filter BP617/73, and the excitation wavelength was 561 nm. For the acquired cell image data, the ratio of the EGFP fluorescence intensity to the mRFP fluorescence intensity (EGFP/mRFP value) was calculated using the image analysis software (CellPathfinder, manufactured by Yokogawa Electric Corporation).

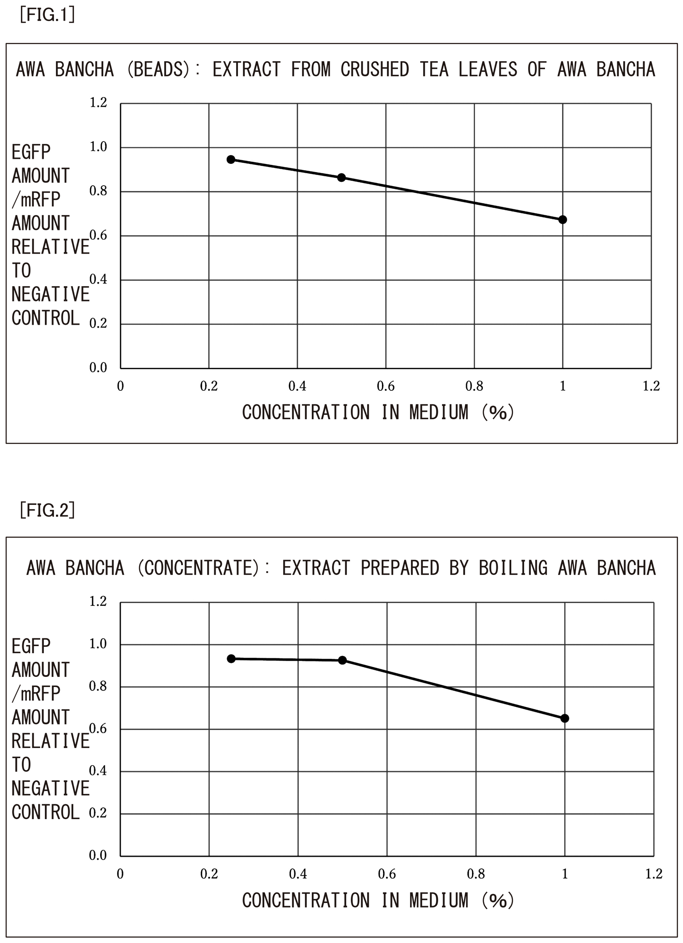

In Figs. 1 to 2, the EGFP/mRFP value in the case of Examples 1 and 2 is expressed as a relative value when the EGFP/mRFP value of the negative control is set to 1. As can be also seen from the figures, it was shown that when the extract from crushed tea leaves of Awa Bancha or the extract prepared by boiling Awa Bancha was added to the medium, the EGFP/mRFP value was decreased, and autophagy activity was enhanced.

<<Preparation Examples>>

(Example 3: Awa Bancha (FD powder): extract from pulverized tea leaves of Awa Bancha)

The Awa Bancha tea leaves used in Example 1 were pulverized with a tabletop mill (Labo Milser LM-PLUS IFM-800, IWATANI CORPORATION) to prepare tea leaf powder. About 10 g of the obtained tea leaf powder was weighed in a beaker, mixed with 10 times the amount (mL) of Milli-Q water, and extracted with hot water at 80°C for 3 hours. After 30% by weight of Diatomaceous earth (Silica 600S, Chuo Silica Co., Ltd.) based on the dry weight of the tea leaves was added by body feed, suction filtration was performed using a Buchner funnel and filter paper. The filtered aqueous solution was powdered using a shelf type freeze drying (FD) machine to obtain a specimen, and the specimen was subjected to the following evaluation of hyaluronic acid production-promoting activity and evaluation of antioxidant activity.

<<Evaluation of Hyaluronic Acid Production-Promoting Activity>>

The specimen was added to normal human adult skin fibroblasts, and the effect on the amount of hyaluronic acid contained in the culture supernatant was evaluated by the following method.

(Cell Culture and Specimen Addition)

Normal human adult skin fibroblasts (KURABO INDUSTRIES, LTD., FC-0024) were subcultured in DMEM (Nacalai Tesque, Inc., 08490-05) supplemented with 10% FBS (Cytiva, SH30396.03) at 37°C under 5%$CO_2$. Cells were seeded in a 96-well plate at $3 \times 10^4$ cells/well (0.1 mL medium) and cultured for 24 hours. The specimen was dissolved in water to be 10 mg/mL, and added to DMEM so that the concentration of the specimen was 0 $\mu$g/mL (solvent control), 12.5 $\mu$g/mL, 25 $\mu$g/mL, and 50 $\mu$g/mL. The medium was removed from the 96-well plate, and 100 $\mu$L of DMEM containing the specimen at various concentrations was added to each well. After 24 hours and 48 hours from the addition, the culture supernatant was collected and stored at -80°C.

(Evaluation of Hyaluronic Acid Amount)

Hyaluronan DuoSet ELISA Kit (R&D Systems, Inc., DY3614-05) was used for quantification of the amount of hyaluronic acid contained in the collected culture supernatant. The absorbance was measured with a plate reader (PerkinElmer, Inc., Enspire) according to the product manual, and the amount of hyaluronic acid contained in the unknown concentration sample was calculated based on a calibration curve created from the absorbance of a dilution series of the known concentration sample.

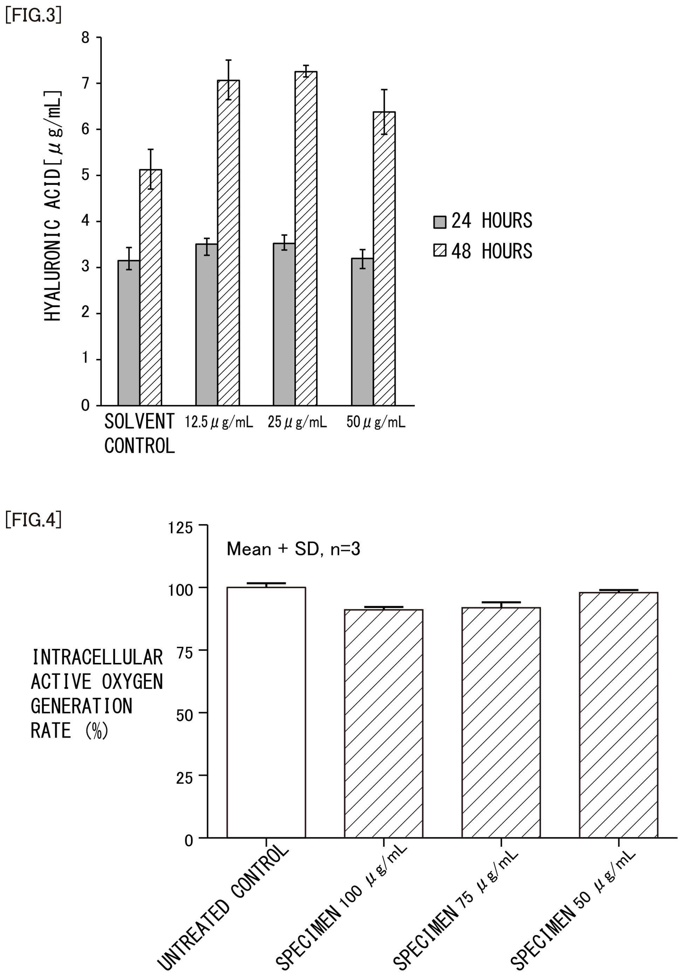

The amounts of hyaluronic acid in the culture supernatants at 24 hours and 48 hours after the replacement with the medium containing a specimen are shown in Fig. 3 (n = 3, mean $\pm$ SD). In Fig. 3, the vertical axis represents the hyaluronic acid amount (unit: $\mu$g/mL), and the horizontal axis represents the specimen addition amount. After 48 hours from the addition of the specimen, the amount of hyaluronic acid in the culture supernatant of the specimen-addition group tended to be larger than that of the solvent control. In the addition groups of 12.5 $\mu$g/mL and 25 $\mu$g/mL, no significant difference was observed after 24 hours, but hyaluronic acid tended to be increased as in the case after 48 hours. From the above, it was suggested that the addition of the specimen to the medium increases hyaluronic acid in the medium, that is, promotes hyaluronic acid production in skin fibroblast cells.

<<Evaluation of Antioxidant Activity>>

Using normal human skin fibroblasts (hereinafter, the cells are referred to as "NHDF cells"), the inhibitory action of the specimen on active oxygen generated in the cells was examined. NHDF cells were purchased from Takara Bio Inc. Fibroblast growth medium 2 (Takara Bio Inc.) was used as a culture medium.

The specimen was dissolved in water to prepare a 50 mg/mL stock test solution. The stock test solution was diluted with a culture medium to prepare test solutions having specimen concentrations of 200, 150, and 100 $\mu$g/mL. NHDF cells were seeded in a 96-well plate and then cultured overnight. Thereafter, each of the test solutions having specimen concentration of 200, 150, and 100 $\mu$g/mL was added thereto, and the cells were cultured at 37°C for 4 hours (the final concentrations of the specimen were 100, 75, and 50 $\mu$g/mL). As an untreated control, the one to which only the medium was added was prepared, and subjected to the test in the same manner. After the culturing, the cells were washed with 1 vol% of a buffer solution (Hanks' Balanced Salt Solution GlutaMAX-1 (100 $\times$)), 50 $\mu$L of CM-$H_2$DCFDA as a reagent that reacts with active oxygen to exhibit fluorescence was added at a concentration of 5 $\mu$mol/L, and the mixture was reacted at 37°C for 10 minutes. Pyocyanin as an intracellular active oxygen generating reagent was added so as to have a final concentration of 100 $\mu$mol/L, and the mixture was reacted at 37°C for 2 hours.

Using a microplate reader [SpectraMax M2e, Molecular Devices, LLC], the fluorescence intensity of the fluorescent substance generated by the reaction between CM-$H_2$DCFDA and active oxygen generated in the cells by the action of pyocyanin was measured (Excitation wavelength: 493 nm, fluorescence wavelength: 523 nm). From the fluorescence intensity of each test solution with respect to the fluorescence intensity of the untreated control, the intracellular active oxygen generation rate was calculated by the following formula. (Reference: Wolfe, KL. et al.: J. Agric. Food Chem., 56, 8418-8426 (2008); Schwarzer, C. et al.: Free Radic. Biol. Med., 45, 1653-1662 (2008))

[Mathematical formula 1]

$$\text{Intracellular active oxygen generation rate (\%)} = \frac{\text{Fluorescence intensity [Sa]}}{\text{Fluorescence intensity [CN] mean}} \times 100$$

Fluorescence intensity [Sa]: fluorescence intensity of each test solution (n = 3)
Fluorescence intensity [CN] mean: mean value of fluorescence intensity of untreated control (n = 3)

The intracellular active oxygen generation rate is shown in Fig. 4 and Table 1 (mean ± SD, n = 3). In Fig. 4, the vertical axis represents the intracellular active oxygen generation rate (unit: %), and the horizontal axis represents the amount of the specimen to be added. The specimen addition group had a lower intracellular active oxygen generation rate than the untreated control, and the intracellular active oxygen generation rate tended to decrease depending on the concentration of the specimen. From the above, it was suggested that the addition of the medium of the specimen decreases the intracellular active oxygen generation rate, that is, suppresses the generation of active oxygen in cells.

[Table 1]

| | Intracellular active oxygen generation rate (%) | | | | |
|---|---|---|---|---|---|
| | n=1 | n=2 | n=3 | Mean value | Standard deviation |
| Untreated control | 99.7 | 98.5 | 101.8 | 100 | 1.7 |
| Specimen 100 $\mu$ g/mL | 92.4 | 90.6 | 90.3 | 91 | 1.1 |
| Specimen 75 $\mu$ g/mL | 91.0 | 90.5 | 94.4 | 92 | 2.1 |
| Specimen 50 $\mu$ g/mL | 96.8 | 98.6 | 98.6 | 98 | 1.0 |

Cross-Reference to Related Applications

The present application claims priority based on Japanese Patent Application No. 2023-088180 filed with the Japan Patent Office on May 29, 2023, the entire disclosure of which is entirely incorporated herein by reference.

**Claims**

1. An autophagy activator comprising a lactic acid fermentation product obtained by fermenting a tea plant-derived raw material with lactic acid bacteria.

2. The autophagy activator according to claim 1, wherein the lactic acid fermentation product is obtained by submerging the tea plant-derived raw material after boiling and/or steaming with the lactic acid bacteria to perform lactic acid fermentation.

3. The autophagy activator according to claim 1, wherein the tea plant-derived raw material is a tea leaf.

4. The autophagy activator according to claim 1, wherein the lactic acid bacteria include at least one selected from Lactobacillus pentosus and Lactobacillus plantarum.

5. The autophagy activator according to claim 1, wherein the lactic acid fermentation product is Awa Bancha.

6. A food, beverage, supplement, cosmetic, pharmaceutical or quasi-pharmaceutical product comprising the autophagy activator according to any one of claims 1 to 5.

7. A cosmetic for skin elasticity improvement, moisture-retention improvement, wrinkle prevention, anti-aging, or age-defying, the cosmetic comprising the autophagy activator according to any one of claims 1 to 5.

8. A supplement for anti-aging, age-defying or lifestyle-disease prevention, the supplement comprising the autophagy activator according to any one of claims 1 to 5.

9. A hyaluronic acid production promoter comprising a lactic acid fermentation product obtained by fermenting a tea plant-derived raw material with lactic acid bacteria.

10. The hyaluronic acid production promoter according to claim 9, wherein the lactic acid fermentation product is obtained by submerging the tea plant-derived raw material after boiling and/or steaming with the lactic acid bacteria to perform lactic acid fermentation.

11. The hyaluronic acid production promoter according to claim 9, wherein the tea plant-derived raw material is a tea leaf.

12. The hyaluronic acid production promoter according to claim 9, wherein the lactic acid bacteria include at least one selected from Lactobacillus pentosus and Lactobacillus plantarum.

13. The hyaluronic acid production promoter according to claim 9, wherein the lactic acid fermentation product is Awa Bancha.

14. A food, beverage, supplement, cosmetic, pharmaceutical or quasi-pharmaceutical product comprising the hyaluronic acid production promoter according to any one of claims 9 to 13.

15. A cosmetic for skin elasticity improvement, moisture-retention improvement, wrinkle prevention, anti-aging, or age-defying, the cosmetic comprising the hyaluronic acid production promoter according to any one of claims 9 to 13.

16. A supplement for anti-aging, age-defying or lifestyle-disease prevention, the supplement comprising the hyaluronic acid production promoter according to any one of claims 9 to 13.

17. An antioxidant comprising a lactic acid fermentation product obtained by fermenting a tea plant-derived raw material with lactic acid bacteria.

18. The antioxidant according to claim 17, wherein the lactic acid fermentation product is obtained by submerging the tea plant-derived raw material after boiling and/or steaming with the lactic acid bacteria to perform lactic acid fermentation.

19. The antioxidant according to claim 17, wherein the tea plant-derived raw material is a tea leaf.

20. The antioxidant according to claim 17, wherein the lactic acid bacteria include at least one selected from Lactobacillus pentosus and Lactobacillus plantarum.

21. The antioxidant according to claim 17, wherein the lactic acid fermentation product is Awa Bancha.

22. A food, beverage, supplement, cosmetic, pharmaceutical or quasi-pharmaceutical product comprising the antioxidant according to any one of claims 17 to 21.

23. A cosmetic for skin elasticity improvement, moisture-retention improvement, wrinkle prevention, anti-aging, or age-defying, the cosmetic comprising the antioxidant according to any one of claims 17 to 21.

24. A supplement for anti-aging, age-defying or lifestyle-disease prevention, the supplement comprising the antioxidant according to any one of claims 17 to 21.

[FIG.1]

AWA BANCHA (BEADS): EXTRACT FROM CRUSHED TEA LEAVES OF AWA BANCHA

EGFP AMOUNT /mRFP AMOUNT RELATIVE TO NEGATIVE CONTROL

CONCENTRATION IN MEDIUM (%)

[FIG.2]

AWA BANCHA (CONCENTRATE): EXTRACT PREPARED BY BOILING AWA BANCHA

EGFP AMOUNT /mRFP AMOUNT RELATIVE TO NEGATIVE CONTROL

CONCENTRATION IN MEDIUM (%)

[FIG.3]

[FIG.4]

## INTERNATIONAL SEARCH REPORT

International application No.

**PCT/JP2024/018958**

| A. | CLASSIFICATION OF SUBJECT MATTER |
|---|---|

*A23L 33/105*(2016.01)i; *A23F 3/30*(2006.01)i; *A23L 2/52*(2006.01)i; *A61K 35/744*(2015.01)i; *A61K 36/82*(2006.01)i; *A61P 43/00*(2006.01)i; *C12N 1/20*(2006.01)i

FI:  A23L33/105; A61P43/00 105; A61K35/744; A23F3/30; A23L2/00 F; A23L2/52; C12N1/20 E; A61K36/82

According to International Patent Classification (IPC) or to both national classification and IPC

| B. | FIELDS SEARCHED |
|---|---|

Minimum documentation searched (classification system followed by classification symbols)

A23L33/105; A23F3/30; A23L2/52; A61K35/744; A61K36/82; A61P43/00; C12N1/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT | |
|---|---|---|

| Category* | Citation of document, with indication, where appropriate, of the relevant passages | Relevant to claim No. |
|---|---|---|
| Y | 池田 絵梨ら, 阿波晩茶の浸出条件が抗酸化活性とカテキン類含量に及ぼす影響, 徳島県立工業技術センター研究報告 = Report of Tokushima Prefectural Industrial Technology Center, January 2022, vol. 30, pp. 11-14, internet:URL<https://agriknowledge.affrc.go.jp/RN/2010941350.pdf>, (IKEDA, Eri et al. Effect of Infusing Condition of Awa-ban cha on Antioxidative Activity and Catechin Content.)<br>1 Introduction, 3 Results and discussion, fig. 1 | 1-8 |
| Y | JP 2020-018294 A (MEIJI CO., LTD.) 06 February 2020 (2020-02-06)<br>claims 1-9, paragraphs [0050], [0052], examples | 1-8 |
| Y | JP 2022-146956 A (NAGAMINE SEICHA CO., LTD.) 06 October 2022 (2022-10-06)<br>claim 1, paragraph [0003], examples | 1-8 |
| Y | WO 2022/015248 A1 (NATIONAL UNIVERSITY OF SINGAPORE) 20 January 2022 (2022-01-20)<br>claims 1-22, examples | 1-8 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 July 2024** | **30 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

International application No.

**PCT/JP2024/018958**

### C.  DOCUMENTS CONSIDERED TO BE RELEVANT

| Category* | Citation of document, with indication, where appropriate, of the relevant passages | Relevant to claim No. |
|---|---|---|
| Y | JP 2021-180619 A (NATIONAL UNIVERSITY CORPORATION CHIBA UNIVERSITY) 25 November 2021 (2021-11-25)<br>claims 1-11, paragraphs [0016]-[0017], [0019] | 1-8 |
| Y | JP 2003-333990 A (ITO EN LTD.) 25 November 2003 (2003-11-25)<br>claims 1-11, paragraph [0012] | 1-8 |
| Y | UCHINO, Masataka et al. Microorganism and Polyphenol in Post Fermented Tea, Awabancha Produced in Tokushima Prefecture. 徳島県産後発酵茶「阿波番茶」の微生物とポリフェノールについて. Food Preservation Science. 2020, vol. 46, no. 2, pp. 63-69, DOI: 10.5891/jafps.46.63<br>Experimental Results and Discussion | 1-8 |
| Y | JIA, Qiang et al. Epigallocatechin-3-gallate attenuates myocardial fibrosis in diabetic rats by activating autophagy. Experimental Biology and Medicine. 14 July 2022, vol. 247, no. 17, pp. 1591-1600, DOI: 10.1177/15353702221110646<br>abstract | 1-8 |
| Y | YAN, Gangli et al. Effects of Vestibular Damage on the Sleep and Expression Level of Orexin in the Hypothalamus of Rats and Its Correlation with Autophagy and Akt Tumor Signal Pathway. Journal of Oncology. 26 June 2022, vol. 2022, pp. 1-11, DOI: 10.1155/2022/2514555<br>abstract | 1-8 |
| Y | HE, Huan et al. Epigallocatechin-3-gallate pretreatment alleviates doxorubicin-induced ferroptosis and cardiotoxicity by upregulating AMPK$\alpha$2 and activating adaptive autophagy. Redox Biology. December 2021, vol. 48, p. 102185, DOI: 10.1016/j.redox.2021.102185<br>abstract | 1-8 |
| A | MI, Xiaojie et al. In vitro assessment of the anti-inflammatory and skin-moisturizing effects of Filipendula palmata (Pall.) Maxim. On human keratinocytes and identification of its bioactive phytochemicals. Journal of Ethnopharmacology. October 2022, vol. 296, p. 115523, DOI: 10.1016/j.jep.2022.115523<br>abstract | 1-8 |

Form PCT/ISA/210 (second sheet) (July 2022)

## INTERNATIONAL SEARCH REPORT

International application No.

**PCT/JP2024/018958**

---

**Box No. III  Observations where unity of invention is lacking (Continuation of item 3 of first sheet)**

This International Searching Authority found multiple inventions in this international application, as follows:

Document 1: 池田 絵梨ら, 阿波晩茶の浸出条件が抗酸化活性とカテキン類含量に及ぼす影響, 徳島県立工業技術センター研究報告 = Report of Tokushima Prefectural Industrial Technology Center, January 2022, vol. 30, pp. 11-14, internet:URL<https://agriknowledge.affrc.go.jp/RN/2010941350.pdf>, (IKEDA, Eri et al. Effect of Infusing Condition of Awa-ban cha on Antioxidative Activity and Catechin Content.)
Document 2: JP 2020-018294 A (MEIJI CO., LTD.) 06 February 2020 (2020-02-06) claims 1-9, paragraphs [0050], [0052], examples (Family: none)
Document 3: JP 2022-146956 A (NAGAMINE SEICHA CO., LTD.) 06 October 2022 (2022-10-06) claim 1, paragraph [0003], examples (Family: none)
Document 4: WO 2022/015248 A1 (NATIONAL UNIVERSITY OF SINGAPORE) 20 January 2022 (2022-01-20) claims 1-22, examples & JP 2023-533956 A & US 2023/0232853 A1 & EP 4181685 A1
Document 5: JP 2021-180619 A (NATIONAL UNIVERSITY CORPORATION CHIBA UNIVERSITY) 25 November 2021 (2021-11-25) claims 1-11, paragraphs [0016]-[0017], [0019] & CN 113678918 A
Document 6: JP 2003-333990 A (ITO EN LTD.) 25 November 2003 (2003-11-25) claims 1-11, paragraph [0012] (Family: none)
Document 7: UCHINO, Masataka et al. Microorganism and Polyphenol in Post Fermented Tea, Awabancha Produced in Tokushima Prefecture. 徳島県産後発酵茶「阿波番茶」の微生物とポリフェノールについて. Food Preservation Science. 2020, vol. 46, no. 2, pp. 63-69, DOI: 10.5891/jafps.46.63

(Invention 1) Claims 1-8
Claim 1 has a special technical feature of "an auto-purge activator containing lactic acid fermentation product obtained by fermenting camellia sinensis-derived raw materials with lactic acid bacteria."
Therefore, claims 1-8 are classified as invention 1.

(Invention 2) Claims 9-16
Claims 9-16 share the common technical feature of "including lactic acid fermented product obtained by fermenting raw materials derived from camellia sinensiswith lactic acid bacteria," with claim 1 classified as invention 1.
However, the technical feature does not make a contribution over the prior art in light of the disclosures of documents 1-7 and thus cannot be the to be a special technical feature.
Also, there are no other the same as or corresponding special technical features between these inventions.
In addition, claims 9-16 are not dependent on claim 1. Furthermore, claims 9-16 are not substantially identical to or similarly closely related to any of the claims classified as invention 1.
Therefore, claims 9-16 cannot be classified as invention 1.
Also, claim 9-16 has a special technical feature of "a hyaluronic acid production accelerator containing lactic acid fermentation product obtained by fermenting raw materials derived from camellia sinensiswith lactic acid bacteria," and thus are classified as invention 2.

(Invention 3) Claims 17-24
Claims 17-24 share, with claims 1-8 classified as invention 1 and claims 9-16 classified as invention 2, the technical feature of "a lactic acid fermentation product obtained by fermenting camellia sinensis-derived raw materials with lactic acid bacteria."
However, the technical feature does not make a contribution over the prior art in light of the disclosures of documents 1-7 and thus cannot be a special technical feature.
Also, there are no other the same as or corresponding special technical features between these inventions.
In addition, claims 17-24 are not dependent on claim 1 or 9. Furthermore, claims 17-24 are not substantially identical to or similarly closely related to any of the claims classified as invention 1 or 2.
Thus, claims 17-24 cannot be classified as either invention 1 or 2.
Also, claims 17-24 have the special technical feature of an "antioxidant containing lactic acid fermented product obtained by fermenting raw materials derived from camellia sinensis with lactic acid bacteria," and are thus classified as invention 3.

---

Form PCT/ISA/210 (continuation of first sheet) (July 2022)

**INTERNATIONAL SEARCH REPORT**

| International application No. |
|---|
| **PCT/JP2024/018958** |

**Box No. III      Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-8**

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/JP2024/018958**

| Patent document cited in search report | | | Publication date (day/month/year) | Patent family member(s) | | | Publication date (day/month/year) |
|---|---|---|---|---|---|---|---|
| JP | 2020-018294 | A | 06 February 2020 | (Family: none) | | | |
| JP | 2022-146956 | A | 06 October 2022 | (Family: none) | | | |
| WO | 2022/015248 | A1 | 20 January 2022 | JP | 2023-533956 | A | |
| | | | | US | 2023/0232853 | A1 | |
| | | | | EP | 4181685 | A1 | |
| JP | 2021-180619 | A | 25 November 2021 | CN | 113678918 | A | |
| JP | 2003-333990 | A | 25 November 2003 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019094303 A

- JP 2023088180 A

**Non-patent literature cited in the description**

- *EMBO Journal*, 2000, vol. 19, 5720-5728
- **KIMURA S. ; NODA T. ; YOSHIMORI T.** Dissection of the Autophagosome Maturation Process by a Novel Reporter Protein, Tandem Fluorescent-Tagged LC3. *Autophagy*, 2007, vol. 3, 452-460
- **SHOTA KOYAMA et al.** Research on microorganisms of post-fermented tea produced in Tokushima. *Awa Bancha*

- **BHARGAVA K.H. et al.** Structural basis for autophagy inhibition by the human Rubicon-Rab7 complex. *Proceedings of the National Academy of Sciences of the United States of America*, 2020, vol. 117, 17003-17010
- **WOLFE, KL et al.** *J. Agric. Food Chem.*, 2008, vol. 56, 8418-8426
- **SCHWARZER, C. et al.** *Free Radic. Biol. Med.*, 2008, vol. 45, 1653-1662